# EUROPEAN PATENT APPLICATION

(11) **EP 2 338 401 A1**
(43) Date of publication of application: **29.06.2011**
(21) Application number: 10015325.3
(22) Date of filing: 06.12.2010
(51) Int. Cl.: A61B 1/00

(54) **Endoscope cover fixing device and fixing system**

(30) Priority: 22.12.2009 JP 2009291500
(71) Applicant: Tyco Healthcare Group, LP, Mansfield, MA 02048 (US)
(72) Inventor: Iwamizu, Keita, Shizuoka 437-0004 (JP); Satoh, Takashi, Shizuoka 437-0004 (JP)
(74) Representative: Olsson, Carl H.S.

(57) **Abstract**

An endoscope cover fixing device able to reliably fix the position of a cover to an endoscope without associated damage. The endoscope cover fixing device includes a fixing device main body and a wedge-shaped portion. Insert portions 43a, 44a into which the endoscope 31 is insertable are formed in the fixing device main body 42. The wedge-shaped portion 46 is provided in the insert holes 43a, 44a of the fixing device main body 42 to protrude to the distal end side of the fixing device main body 42. The wedge-shaped portion 46 is inlayed through a proximal end 23 side of the cover 21 into a gap between the endoscope 31 and the cover 21.

## Description

### FIELD OF THE INVENTION

The present invention generally relates to an endoscope cover fixing device for, when a cover having a closed distal end is placed over an endoscope, fixing the position of the cover on the endoscope, and to a system employed for fixing the cover to the endoscope.

### BACKGROUND OF THE INVENTION

By necessity, medicine requires the diagnosis and treatment of interior body cavities that are not otherwise visible, and endoscopes are a well-known conventional medical instrument for implementing such diagnoses in a comparatively simple manner. Common endoscope structures comprise a lens portion provided in the distal end of a tube of a size that is insertable in an interior body cavity, and images captured by this lens portion are transmitted by way of optical fibers arranged within the tube.

Viscous body fluids and impurities affix to the outer circumferential surface of an endoscope during use. Accordingly, endoscopes must be thoroughly cleansed and disinfected after use to prevent the transfer of infectious diseases and the like. However, the complicated and time-consuming nature of the endoscope cleansing and disinfecting operations affects the operability thereof. A further problem pertains to the accelerated deterioration of the endoscope caused by the regular cleansing and disinfecting thereof.

With this in mind, the use of a sheath-like, contamination-preventing endoscope cover which has a closed distal end and which is placed over the endoscope to prevent contamination of the endoscope and is discarded after use has been recently considered.

However, the acquisition of satisfactory images with a cover placed over the endoscope requires that the cover be fitted closely to the lens portion in the distal end of the tube. However, with covers of this type, dimensional variations in the manufacturing process thereof are unavoidable. Such variations often render it difficult to fit the cover closely to the lens portion of the tube.

With this in mind, structures for fixing a cover to an endoscope have been hitherto proposed (for example, see Japanese Patent No. 335932). In the cover-type endoscope as described in the '932 Japanese patent, an engagement portion is provided in the inner circumference of the proximal end side of the cover, and a slidable latch portion corresponding to the engagement portion is provided in the outer circumference of an operating portion of the endoscope. According to this configuration, friction between the latch portion and the engagement portion causes the latch portion to engage closely with the engagement portion which, in turn, facilitates fixing of the position of the cover on the endoscope.

However, the close engagement and fixing of a cover to an endoscope necessitates the employment of components that enhance the rigidity of the cover itself, and that increase the friction force in the conventional art as described above. The employment of these components increases the complexity of the machining and the assembly of the device, and also increases manufacturing and other costs.

Thereupon, the employment of a fixing device for positioning and fixing a cover to an endoscope configured separately to the endoscope and the cover such as a conventional medical clamp for example, was briefly considered. However, the employment of a conventional medical clamp comprises clamping the cover and the endoscope together with a pair of clamping portions and locking them in the clamped state. However, there is a significant pressing force or pressure exerted on the endoscope in the radial direction. This increases the likelihood that the image guide or light guide of the endoscope, usually constituted from optical fibers, will be damaged.

### SUMMARY OF THE INVENTION

With the foregoing conditions in mind, it is an object of the present invention to provide an endoscope cover fixing device and fixing system able to reliably fix the position of a cover on an endoscope without associated endoscope damage.

In general, this invention is directed to an endoscope cover fixing device employed for, when a cover having a closed distal end is placed over an endoscope, fixing the position of the cover on the endoscope. The device comprises a fixing device main body having an insert portion into which the endoscope is insertable. The device further comprises a wedge-shaped portion in the insert portion of the fixing device provided to protrude to a distal end side of the fixing device main body. The wedge-shaped portion is inlayed through a proximal end side of the cover into a gap between the endoscope and the cover.

The wedge-shaped portion inlayed through the proximal end side of the cover into the gap between the endoscope and the cover affords the expansion of this gap and absorbs the reaction forces from an endoscope tube. The endoscope tube has an elastic body. In addition, the reaction forces are used to fix the position of the cover on the endoscope. The wedge-shaped portion exerts an appropriate pressing force on the endoscope during the fixing operation, thereby avoiding exertion of an excessive pressing force on a specific region of the endoscope in the radial direction. Accordingly, the cover is able to be reliably fixed in position on the endoscope without associated endoscope damage.

According to an embodiment of the invention, the endoscope cover fixing device is characterized in that the insert portion comprises a plurality of linearly arranged insert portions formed in different positions spaced apart in the fixing device main body. The wedge-shaped portion is provided in a circumferential edge of the insert portion of the plurality of insert positions that is not located at the frontmost end side of the fixing device main body.

Insertion of the endoscope and the cover in the plurality of insert positions having the positional relationship described above affords, to some extent, the positioning of the endoscope and the cover with respect to the wedge-shaped portion. Accordingly, the operation for inlaying the wedge-shaped portion into the gap between the endoscope and the cover is made easier.

According to an embodiment of the invention, the fixing device main body constitutes an annular member having a front wall portion and a rear wall portion disposed opposing and apart from the aforementioned front wall portion. The insert portion comprises two insert holes which are formed in the front wall portion and the rear wall portion respectively, and which are disposed linearly on a common axial line. The wedge-shaped portion is provided in a circumferential edge portion of the insert hole formed in the aforementioned rear wall portion.

According to an embodiment of the invention, the insertion of the endoscope and the cover in the two insert holes having the positional relationship described above affords, to some extent, the positioning of the endoscope and the cover with respect to the wedge-shaped portion. Accordingly, the operation for inlaying the wedge-shaped portion between the endoscope and the cover is made easier. In addition, because the fixing device main body is an annular member, the rigidity of the fixing device as a whole is able to be enhanced. Moreover, because the adopted structure is one in which the wedge-shaped portion is disposed in an interior region of the annular member, stable fixing of the position of the cover is achieved.

The wedge-shaped portion is a cylindrical or hollow truncated cone shape having a tapered portion in the outer circumferential side. The length of the wedge-shaped portion is established so as to be greater than the pitch of a coil disposed in an over-tube of the endoscope.

According to an embodiment of the invention, because the wedge-shaped portion is a cylindrical or hollow truncated cone shape having a tapered portion in the outer circumferential side, the wedge-shaped portion is easily inlayed in the gap between the endoscope and the cover. Although the inner circumferential surface of the wedge-shaped portion is in contact with the outer circumferential surface of the endoscope at this time, because the length of the contact section thereof is greater than the pitch of the coil, the pressing force is dispersed rather than concentrated on a specific region of the outer circumferential surface of the endoscope. Accordingly, damage to the endoscope is more reliably prevented.

Aspects of the invention are further directed to an endoscope cover fixing system for, when a cover having a closed distal end is placed over an endoscope, employing a fixing device to fix the position of the cover on the endoscope. The fixing device includes a wedge-shaped portion, and the cover is fixed in position as a result of the inlay of the wedge-shaped portion through the proximal end side of the cover into the gap between the endoscope and the cover.

According to an embodiment of the invention, the wedge-shaped portion inlayed between the endoscope and the cover expands the gap therebetween and absorbs the reaction forces of the endoscope tube. The endoscope tube has an elastic body. In addition, these reaction forces are used to fix the position of the cover on the endoscope. The wedge-shaped portion exerts an appropriate pressing force on the endoscope during the fixing operation without exerting an excessive pressing force on a specific region of the endoscope in the radial direction. Accordingly, the cover is able to be reliably fixed in position on the endoscope without associated endoscope damage.

Other objects and features will be in part apparent and in part pointed out hereinafter.

### BRIEF DESCRIPTION OF THE DRAWINGS

FIG. 1 is a schematic diagram of an endoscope cover fixing device, an endoscope and a cover of a first embodiment of the present invention;

FIG. 2(a) is a front view of the endoscope cover fixing device of the first embodiment;

FIG. 2(b) is a cross-sectional view of the endoscope cover fixing device of FIG. 1;

FIG. 2(c) is a left-side view of the endoscope cover fixing device of FIG. 1;

FIG.2(d) is a right-side view of the endoscope cover fixing device of FIG. 1;

FIG. 3 is an enlarged, fragmentary cross-sectional view illustrating the endoscope cover fixing device during the fixing operation;

FIG. 4 is a front view of an endoscope cover fixing device of a second embodiment of the present invention;

FIG. 5 is a cross-sectional view of an endoscope cover fixing device of a third embodiment of the present invention;

FIG. 6 is a cross-sectional view of an endoscope cover fixing device of a fourth embodiment of the present invention;

FIG. 7 is a front view of an endoscope cover fixing device of another embodiment of the present invention;

FIG. 8 is a cross-sectional view of an endoscope cover fixing device of yet another embodiment of the present invention;

FIG. 9 is a front view of an endoscope cover fixing device of yet another embodiment of the present invention;

FIG. 10 is a front view of an endoscope cover fixing device of yet another embodiment of the present invention; and

FIG. 11 is a front view of an endoscope cover fixing device of yet another embodiment of the present invention.

### REFERENCE CHARACTERS

21 (Endoscope) Cover

31 Endoscope

32 Over-tube

33 Coil

41, 51, 61, 71, 81, 91, 101, 111, 121 (Endoscope cover) fixing device

42, 42A, 42B, 42C Fixing device main body

43a, 44a, 72a Insert hole constituting insert portion

46, 52, 82, 92 Wedge-shaped portion

43 Front-wall portion

44 Rear-wall portion

P1 Pitch

T1 Tapered portion

Corresponding reference characters indicate corresponding parts throughout the drawings.

### DESCRIPTION OF THE PREFERRED EMBODIMENTS

[First Embodiment]

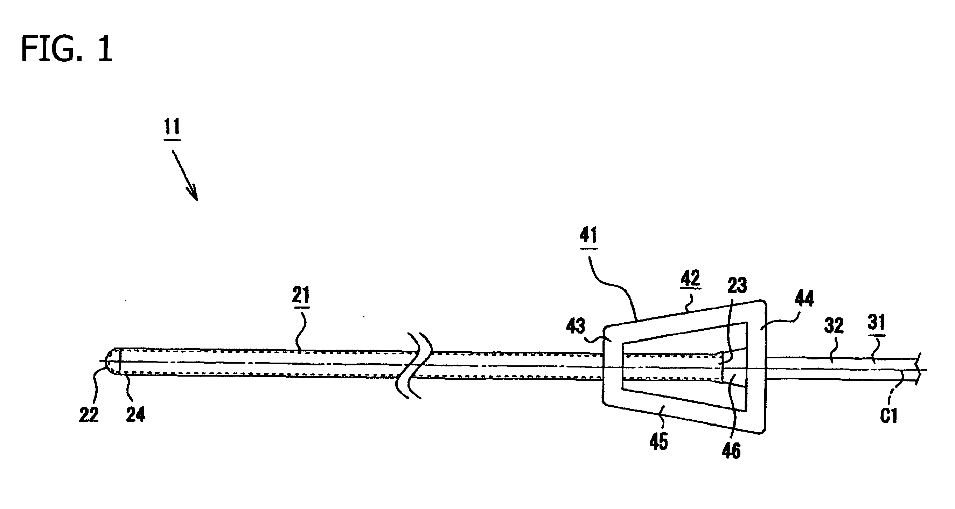

An endoscope cover fixing device of a first embodiment of the present invention will be hereinafter described with reference to FIGS. 1 to 3.

FIG. 1 schematically shows an endoscope set 11 comprising an endoscope cover fixing device 41, an endoscope 31 and an endoscope cover 21 of the first embodiment. The endoscope 31 comprises a lens portion (not shown) provided at a distal end of a flexible cover tube 32 of a diameter suitable for insertion into an interior body cavity. In a non-limiting embodiment, endoscope 31 is comparatively small and has a maximum diameter of approximately 3mm. The endoscope cover 21 constitutes a sheath-like member formed from a flexible and light-permeable material such as a synthetic resin, and has a slightly larger internal diameter than the endoscope 31. A distal end 22 of the endoscope cover 21 is rounded and closed. The cover 21 is placed over the endoscope 31 and, in this state, the inner surface of the distal end 22 of the cover 21 fits closely to the lens portion.

The endoscope cover fixing device 41 constitutes a member for fixing the position of the cover 21 on the endoscope 31, and is mounted at the proximal end 23 of the cover 21.

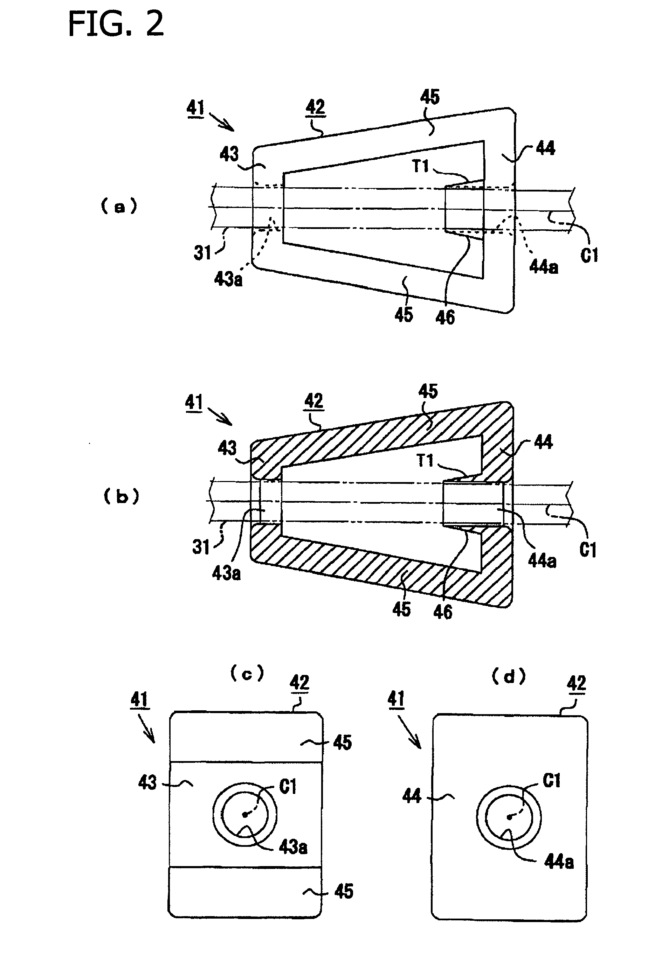

As illustrated in FIG. 1 and FIG. 2, the endoscope cover fixing device 41 includes a fixing device main body 42. The fixing device main body 42 constitutes an annular member which includes a front-wall portion 43, a rear-wall portion 44, and a pair of coupling portions 45. The rear-wall portion 44 is fabricated to be longer than the front-wall portion 43, and is disposed opposing and apart from the front-wall portion 43. The rear-wall portion 44 and the front-wall portion 43 describe a mutually parallel positional relationship. The pair of coupling portions 45 couple the two ends of the rear-wall portion 44 to the two ends of the front-wall portion 43. As a result, the fixing device main body 42 describes a substantially trapezoidal shape in the front view. The coupling portions 45 of the fixing device main body 42 angle outwardly from the front-end side to the rear-end side.

An insert hole 43a of a circular cross-section is formed in a centre portion of the front-wall portion 43 in such a way as to pass through the inner and outer surfaces of the front-wall portion 43. The front-end side insert hole 43a has a circular cross-section, and the inner diameter thereof is set to a size at which the endoscope 31, in a state in which the cover 21 is placed thereover, is insertable therein. Accordingly, the inner diameter of the insert hole 43a is set to be slightly larger than the outer diameter dimension of the cover 21. Similarly, an insert hole 44a serves as an insert portion, preferably of a circular cross-section, and is formed in the centre portion of the rear-wall portion 44 in such a way as to pass through the inner and outer surfaces of the rear-wall portion 44. In other words, the two insert holes 43a, 44a are disposed linearly on a common axial line C1 of the fixing device 41, and the centre of the two insert holes 43a, 44a preferably lies on the common axial line C1.

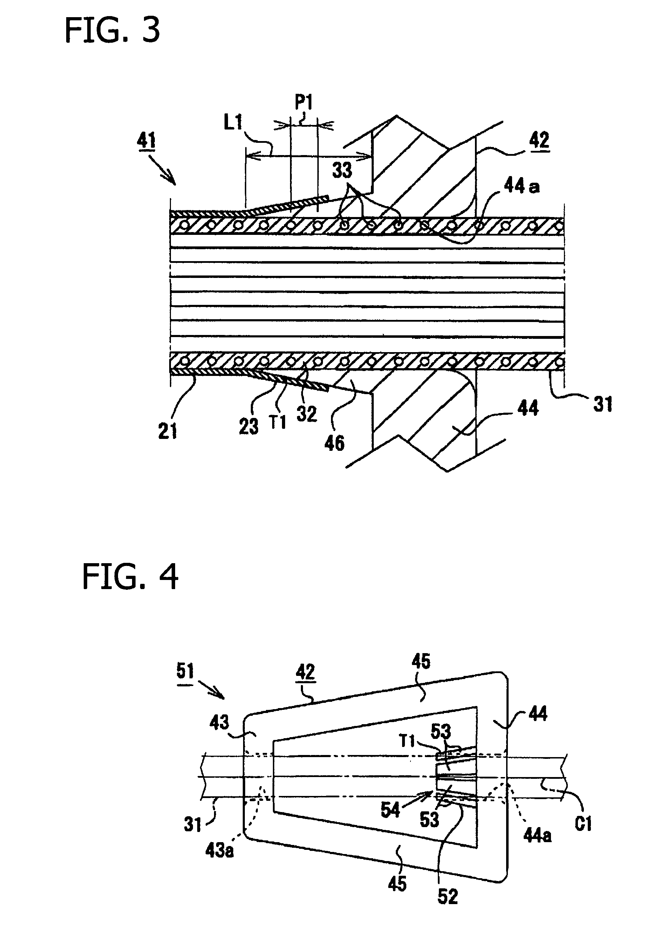

A wedge-shaped portion 46 is provided in the circumferential edge portion of the insert hole 44a so as to protrude to the front-end side thereof. The wedge-shaped portion 46 describes a hollow truncated cone shape having a tapered portion T1 in its outer circumference that expands in the direction of the proximal end side. The outer circumferential surface of the wedge-shaped portion 46 may be rough-surface machined. As illustrated in FIG. 3, a length L1 of the wedge-shaped portion 46 is preferably set to be at least several times greater than a pitch P1 of a coil 33 disposed in an over-tube 32 of the endoscope 31. For example, if the pitch P1 of the coil 33 is approximately 0.6mm, the length L1 may be set to the order of 5mm to 20mm.

The inner diameter of the wedge-shaped portion 46 (i.e., inner diameter of the insert hole 44a) is set to a value of the same order as the outer diameter dimension of the endoscope 31. Additionally, the minimum outer diameter dimension of the wedge-shaped portion 46 is set to a value of the same order as the inner diameter dimension of the cover 21.

The endoscope cover fixing device 41 may be manufactured by injection molding or the like using, for example, a synthetic resin material. The fixing device main body 42 and the wedge-shaped portion 46 of the fixing device 41 may be integrally formed from a single synthetic resin material. Provided the material is flexible, there are no particular restrictions to the synthetic resin material able to be used for the endoscope cover fixing device 41, and various materials, including polypropylene, polycarbonate and nylon, may be employed. In practical design terms, the synthetic resin material selected as the material of the fixing device 41 is preferably harder than at least the cover 21 and the over-tube 32.

A method using the endoscope cover fixing device 41 of this first embodiment will be hereinafter described.

Prior to placing the cover 21 over the endoscope 31, the proximal end 23 side of the cover 21 is inserted into the front-end side insert hole 43a. Next, the distal end portion of the endoscope 31 is inserted through the rear-end side insert hole 44a, and is guided inward from the proximal end 23 of the cover 21. A lens portion of the distal end portion of the endoscope 31 is fitted closely to the inner surface of the distal end 22 of the cover 21. As a result of the movement of the proximal end 23 toward the rear-wall portion 44, the distal end of the wedge-shaped portion 46 is inlayed through the proximal end 23 side of the cover 21 into the gap between the endoscope 31 and the cover 21 (see FIGS. 1 and FIG. 3). At this time, the wedge-shaped portion 46 exerts a force for expanding this gap on the over-tube 32, and absorbs the reaction forces from the over-tube 32. Over-tube 32 is preferably constituted by an elastic member. As a result of these reaction forces, the cover 21 is fixed in position with respect to the endoscope 31.

The cover 21 is positioned with respect to the endoscope 31 by the reaction forces produced when the wedge-shaped portion 46 is inlayed into the gap as described above. During the fixing operation, the wedge-shaped portion 46 is in surface contact with the entire outer circumferential surface of the endoscope 31, and it exerts an appropriate pressing force on the outer circumferential surface of the endoscope 31.In other words, this approach avoids exertion of an excessive pressing force on any specific region of the endoscope 31. Accordingly, the cover 21 is able to be reliably fixed in position with respect to the endoscope 31 without associated damage to the endoscope 31.

Insert holes 43a, 44a are formed in the front-wall portion 43 and rear-wall portion 44 respectively, and are disposed linearly on a common axial line C1. In addition, the wedge-shaped portion 46 is provided in the circumferential edge portion of the insert hole 44a formed in the rear-wall portion 44. Accordingly, the insertion of the cover 21 in the insert hole 43a and the insertion of the endoscope 31 in the insert holes 43a, 44a affords the positioning of the endoscope 31 and the cover 21 with respect to the wedge-shaped portion 46. Consequently, the operation for inlaying the wedge-shaped portion 46 into the gap between the endoscope 31 and the cover 21 is made easier. In addition, because the fixing device main body 42 is an annular member, the rigidity of the fixing device 41 as a whole is increased. Because the wedge-shaped portion 46 constitutes a structure which is disposed in an interior region of an annular member, a protected state is established in which the inlay region is enclosed by the fixing device main body 42. Accordingly, unlike a structure in which the wedge-shaped portion 46 is disposed in an exterior region of an annular member, stable positional fixing can be achieved. Moreover, the overall simplified structure of the fixing device 41 of this embodiment affords a reduction in manufacturing costs.

Because the fixing device 41 of this embodiment comprises a wedge-shaped portion 46 of a hollow truncated cone shape having a tapered portion T1 in its outer circumferential side, the wedge-shaped portion 46 is inlayed easily into a gap between the endoscope 31 and the cover 21. While the inner circumference of the wedge-shaped portion 46 is in contact with the outer circumferential surface of the endoscope 31 at this time, the length of this contacting section is much larger than the pitch P1 of the coil 33. Accordingly, the pressing force is dispersed rather than concentrated on a specific region of the outer circumferential surface of the endoscope 31. Consequently, damage to the endoscope 31 is prevented.

[Second Embodiment]

A second embodiment of the present invention will be hereinafter described with reference to FIG. 4.

As shown in FIG. 4, a fixing device 51 of the second embodiment differs from the fixing device of the first embodiment in respect of the shape of a wedge-shaped body 52. Even though the wedge-shaped body 52 describes a hollow truncated cone shape, it is partitioned in the radial direction by a plurality of slits 54. Accordingly, the wedge-shaped body 52 is constituted from a plurality of partitioned pieces 53. In this configuration as well, the cover 21 is able to be reliably fixed in position on the endoscope 31 without associated damage.

[Third Embodiment]

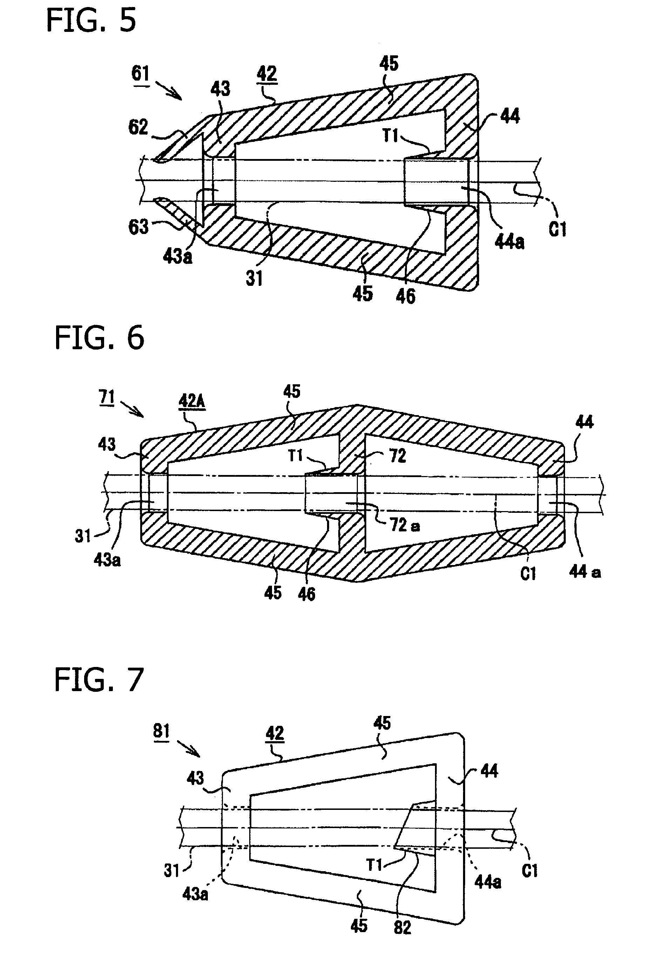

A third embodiment of the present invention will be hereinafter described with reference to FIG. 5.

As shown in FIG. 5, a pair of protrusion pieces 62, 63 are provided in the outer circumferential surface of the front-wall portion 43 in two opposing positions about the insert hole 43a. Accordingly, when the cover 21 is inserted in the insert hole 43a during the fixing operation, the distal ends of these protrusion pieces 62, 63 contact the outer circumferential surface of the cover 21. As a result, the cover 21 is temporarily supported in a way that prevents the fallout thereof from the insert hole 43a. This configuration advantageously makes it easier to inlay the wedge-shaped portion 46 into the gap between the endoscope 31 and the cover 21.

[Fourth Embodiment]

A fourth embodiment of the present invention will be hereinafter described with reference to FIG. 6.

In the fixing device 71 of the fourth embodiment as illustrated in FIG. 6, a fixing device main body 42A constitutes a structure that differs markedly from the structure of the first embodiment and is arrived at by coupling two of the fixing device main bodies 42 of the first embodiment. The fixing device main body 42A of the fixing device 71 comprises, in addition to the front-wall portion 43 and the rear-wall portion 44, a centre wall portion 72. An insert hole 72a constituting an insert portion is formed in the centre wall portion 72, and three insert holes(i.e., the insert hole 72a and other insert holes 43a, 44a) are linearly arranged on a common axial line C1. In addition, in the fourth embodiment, a wedge-shaped portion 46 is provided in the circumferential edge portion of the insert hole 72a which constitutes the second insert hole from the front-end side.

[Other Embodiments]

Having described the invention in detail, it will be apparent that modifications and variations are possible without departing from the scope of the invention defined in the appended claims.

In the aforementioned embodiments the distal end open portion of the wedge-shaped portion 46 describes a shape cut in a plane orthogonal to the axial line of the insert hole 44a. In addition thereto, as in the fixing device 81 of another embodiment as illustrated in FIG. 7, a wedge-shaped portion 82 having an open shape in the distal end is obtained by cutting in a plane diagonally intersecting the axial line of the insert hole 44a (a bevel-cut wedge-shaped portion 82). When a shape such as this is adopted, inlaying of the wedge-shaped portion 82 into the gap is easily achieved.

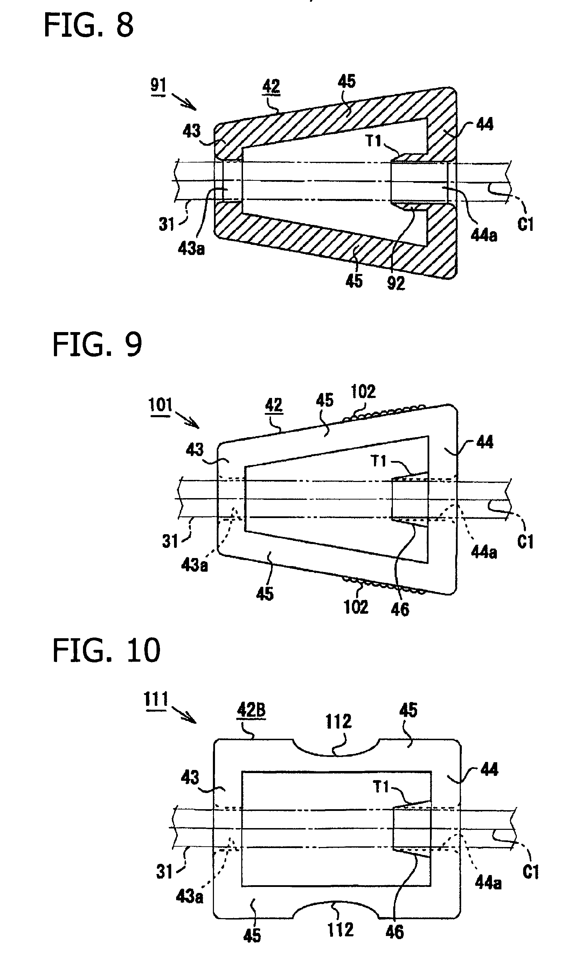

While a tapered portion T1 is provided in the entire outer circumference of the wedge-shaped portion 46 in the aforementioned embodiments, the fixing device 91 of another embodiment as shown in FIG. 8 alternatively illustrates a wedge-shaped portion 92 of a substantially cylindrical shape in which a tapered portion T1 is provided only in one portion of the outer circumference of the wedge-shaped portion 92 (distal end) may be adopted.

As illustrated in a fixing device 101 of another embodiment shown in FIG. 9, a plurality of projecting portions 102 for slip prevention may be provided in the outer circumferential side of the pair of coupling portions 45 of the fixing main body 42. In this configuration, because slippage of the fixing device 101 is unlikely as a result of clasping of the projecting portions 102, operability during the inlay operation is improved. In addition to the projecting portions 102, the outer circumferential surface of the fixing device main body 42 may be machined (for example, rough machined) in order to prevent slippage.

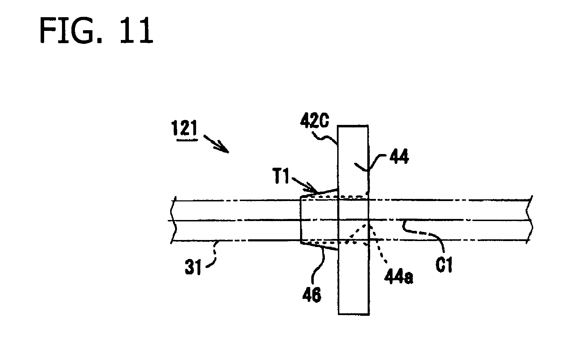

While the shape of the fixing device main body 42 of the embodiments in the front view is of a substantially trapezoidal shape, the configuration is not limited thereto, and other shapes may be adopted. For example, in a fixing device 111 of an embodiment as illustrated in FIG. 10, the shape of a fixing device main body 42B in the front view is substantially rectangular. As described above, a pair of clasping recess portions 112 appropriately sized for insertion of fingertips for gripping are provided in the fixing device main body 42B. In a fixing device 121 of another embodiment shown in FIG. 11, the shape of a fixing device main body 42C is further simplified, and the front-wall portion 43 and pair of coupling portions 45 are omitted altogether.

While the fixing device main bodies 42, 42A, 42B, 42C and wedge-shaped portions 46, 52, 82, 92 of the aforementioned embodiments may be integrally formed from identical synthetic resin materials, the configurations thereof are not limited thereto and these component parts may be formed separately and then joined by some other means (for example, adhesive-based adhesion or thermal welding or the like). Although a synthetic resin material is selected as the material for forming the fixing devices of the aforementioned embodiments, a metal material such as stainless steel or aluminum may also be selected for forming some or all of the components of the fixing device.

In an alternative embodiment, instead of a pair of protrusion pieces 62, 63, either one of these protrusion pieces alone may be provided. The position in which these slip-preventing structures are provided is not limited to the outer circumferential side of the front-wall portion 43 and, for example, they may be provided on the inner surface side of the front-wall portion 43 or the inner surface side of the coupling portions 45. Furthermore, the shape of the slip-preventing structure is not limited to the shape shown in FIG. 5, and other shapes are indeed within the scope of the invention.

The embodiments described above specify the use of insert holes 43a, 44a, 72a constituted as insert portions. In addition or in alternative, notched portions or the like (formed by partial notching) in the width direction, for example, the fixing device main body 42 may also be provided. Notably, provided the fixing device 41 is able to be inserted therein, these holes may be of any suitable shape.

While the embodiments described above describe the cover 21 as fixed in position on the medical endoscope 31, the application thereof in other medical instruments (including industrial endoscopes) is also possible.

In any of the aforementioned embodiments, the length of the aforementioned wedge-shaped portion can be limited to being no less than 0.6mm.

In any of the aforementioned embodiments, a fallout-prevention structure having contact with the cover can be provided in the fixing device main body to prevent fallout of the cover inlayed in the inlay portion.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As various changes could be made in the above constructions, products, and methods without departing from the scope of the invention, it is intended that all matter contained in the above description and shown in the accompanying drawings shall be interpreted as illustrative and not in a limiting sense.

## Claims

1. An endoscope cover fixing device employed for, when a cover having a closed distal end is placed over an endoscope, fixing the position of the cover on the endoscope, said device comprising:
a fixing device main body having an insert portion into which said endoscope is insertable; and
a wedge-shaped portion provided in said insert portion of said fixing device main body, said wedge-shaped portion protruding to a distal end side of said fixing device main body,
wherein said wedge-shaped portion is inlayed through a proximal end side of the cover into a gap between the endoscope and the cover.

2. The endoscope cover fixing device according to claim 1, wherein the insert portion comprises a plurality of linearly arranged insert portions formed in different positions spaced apart in the fixing device main body, wherein the wedge-shaped portion is provided in a circumferential edge of the insert portion of said plurality of insert portions, said circumferential edge not located at a frontmost end side of the fixing device main body.

3. The endoscope cover fixing device according to claim 1, wherein the fixing device main body comprises an annular member having a front wall portion and a rear wall portion disposed opposing and apart from said front wall portion, wherein the insert portion comprises two insert holes, wherein one insert hole of the two insert holes is formed in said front wall portion and the other insert hole of the two insert holes is formed in said rear wall portion, said two insert holes disposed linearly on a common axial line, wherein the wedge-shaped portion is provided in a circumferential edge portion of the insert hole formed in said rear wall portion.

4. The endoscope cover fixing device according to any of Claims 1 to 3, wherein the wedge-shaped portion is a cylindrical or hollow truncated cone shape having a tapered portion in the outer circumferential side, and wherein a length of the wedge-shaped portion is greater than a pitch of a coil disposed in an over-tube of the endoscope.

5. An endoscope cover fixing system for, when a cover having a closed distal end is placed over an endoscope, employing a fixing device to fix a position of the cover on the endoscope, said fixing device comprising a wedge-shaped portion, wherein inlay of said wedge-shaped portion through a proximal end side of the cover into a gap between the endoscope and the cover results in fixing of the position of the cover on the endoscope.
